# FASCICULE DE BREVET EUROPEEN

(11) **EP 2 790 643 B1**
(45) Date de publication et mention de la délivrance du brevet: **19.07.2017**
(21) Numéro de dépôt: 12816700.4
(22) Date de dépôt: 10.12.2012
(51) Int. Cl.: A61K 8/06, A61Q 17/04, A61K 8/02, A61Q 1/02

(54) **METHODE DE SOIN ET/OU DE MAQUILLAGE DE LA PEAU PROCURANT UNE PROTECTION VIS-A-VIS DES RAYONS UV.**
HAUTPFLEGE- UND/ODER SCHMINKVERFAHREN MIT SCHUTZ VOR UV-STRAHLUNG
SKINCARE AND/OR MAKE-UP METHOD PROVIDING PROTECTION AGAINST UV RAYS

(30) Priorité: 12.12.2011 FR 1161461
(43) Date de publication de la demande: 22.10.2014
(73) Titulaire: LvmH Recherche, 45800 Saint-Jean De Braye (FR)
(72) Inventeur: ALARD, Valérie, F-45000 Orleans (FR); BEAUFRERE-SERON, Béatrice, F-45160 Olivet (FR); PERRIER, Eric, F-38138 Les Cotes D'arey (FR)
(74) Mandataire: Mena, Sandra
(86) Numéro de dépôt international: PCT/FR2012/052860
(87) Numéro de publication internationale: WO 2013/088046

(56) Documents cités:
- DATABASE GNPD [Online] MINTEL; mai 2010 (2010-05), "1-2-3 Step Whitening Value Set", XP002680800, Database accession no. 1321698
- DATABASE GNPD [Online] MINTEL; mai 2010 (2010-05), "UV Special Care Set", XP002680801, Database accession no. 1322879
- DATABASE GNPD [Online] MINTEL; octobre 2010 (2010-10), "Globe-Trotter Travel Vanity Case", XP002680802, Database accession no. 1435703
- DATABASE GNPD [Online] MINTEL; août 2011 (2011-08), "The Illuminating Powder", XP002681696, Database accession no. 1618634
- DATABASE GNPD [Online] MINTEL; avril 2010 (2010-04), "The SPF 30 UV Protecting Fluid", XP002681697, Database accession no. 1428056
- DATABASE GNPD [Online] MINTEL; juillet 2008 (2008-07), "Baby's Outing Pack", XP002680803, Database accession no. 950861
- DATABASE GNPD [Online] MINTEL; septembre 2007 (2007-09), "Powder Prickly Heat", XP002681698, Database accession no. 766168

## Description

L'invention a pour objet un produit cosmétique de soin de la peau comprenant au moins deux compositions particulières selon la revendication 1 que l'on applique l'une sur l'autre. Cette méthode d'application permet de concilier une protection quotidienne vis-à-vis des UV et de bonnes propriétés sensorielles, telles que l'absence de sensation de collant ou de gras à l'application.

### ETAT DE L'ART

L'efficacité d'une composition solaire pour la peau se traduit habituellement par un indice de protection ou un facteur de protection solaire (abrégé SPF). Le SPF est défini par le rapport de la quantité d'énergie nécessaire pour provoquer un début d'érythème sur la peau protégée par un agent filtrant les radiations UV, et de la quantité d'énergie nécessaire pour provoquer un début d'érythème sur la peau non protégée. Plus la valeur de SPF mesurée augmente, plus le niveau de protection conférée par le produit vis-à-vis des rayonnements ultraviolets est élevé. Pour atteindre des niveaux d'efficacité significatifs, l'homme de l'art est couramment amené à introduire les agents photoprotecteurs en quantité importante et ce d'autant plus que le niveau d'efficacité requis est élevé.

D'une manière générale, les compositions cosmétiques destinées à la photoprotection de la peau contiennent des filtres UV organiques et/ou inorganiques qui fonctionnent selon leur nature chimique et leurs propriétés physiques, par absorption, réflexion ou diffusion du rayonnement UV. Il existe des produits de protection solaire proprement dits à fort indice de protection, particulièrement indiqués pour des expositions longues et intenses au soleil, souvent de caractère occasionnel. Ces produits solaires existent notamment sous forme de lait, de crème, de spray, de stick et de lingettes imprégnées. Ces produits peuvent permettre d'atteindre des niveaux de photoprotection très élevés.

Outre, les risques de brûlures, on a pu mettre en évidence que le rayonnement ultraviolet provoque le vieillissement extrinsèque de la peau, lié à une altération progressive des fonctions cellulaires consécutive à la dégradation des protéines de la cellule telles que des protéines constitutives de la matrice extracellulaire et des enzymes participant au maintien de l'homéostasie cellulaire. Les ultraviolets provoquent notamment la formation de radicaux libres qui agissent sur les structures susmentionnées, et provoquent inflammation et irritation.

De ce fait, le consommateur souhaite se protéger des effets néfastes des ultraviolets tout au long de la journée et tout au long de l'année, et non plus uniquement pendant les périodes de fort ensoleillement ou en cas d'exposition prolongée au soleil.

On a donc introduit des filtres solaires dans des produits de soin ou de maquillage à usage plus régulier, en particulier quotidien, pour protéger la peau d'un ensoleillement plus faible en intensité et en durée, car cette exposition de faible intensité s'est révélée être un facteur de vieillissement cutané. Parfois, ces compositions contiennent des quantités de filtres UV plus faibles, mais ces filtres nuisent tout de même aux qualités de toucher, de texture et d'application recherchées par le consommateur. Il existe ainsi sur le marché de nombreux produits de soin ou de maquillage, qui comprennent des filtres contre le rayonnement ultraviolet (encore dénommés « filtres UV » dans la présente demande), qui sont soit des filtres physiques (filtres UV minéraux) soit des filtres UV chimiques (filtres UV organiques).
Le document Mintel, 1-2-3 Step Whitening Value Set, décrit un kit comprenant une composition hydratante de SPF 15 sous forme d'émulsion ainsi qu'une poudre anhydre blanchissante de la peau de SPF 18 comprenant une phase huileuse et un filtre UV, sans que la proportion des différents ingrédients soit indiquée. Les filtres solaires présents dans les compositions sont des filtres liposolubles (éthylhexyl méthoxycinnamate).

### BUTS DE L'INVENTION

Un des objectifs du formulateur de produits cosmétiques de soin ou de maquillage est de concilier la quantité de filtres solaires introduits dans le produit, la protection qu'assure le produit vis-à-vis des UV, et les propriétés sensorielles du produit. Le produit cosmétique de l'invention permet d'améliorer la protection de la peau vis-à-vis des UV sans augmenter la quantité de filtres UV nécessaire pour obtenir cette protection, et par conséquent en conservant des propriétés sensorielles satisfaisantes pour les produits appliqués sur la peau. Le produit cosmétique de l'invention permet également de proposer un produit de soin, un produit de maquillage ou un produit qui remplit à la fois les fonctions de soin et de maquillage de la peau dont la protection vis-à-vis des UV est comparable à celles des produits de soin et de maquillage de l'art antérieur, et dont les propriétés sensorielles sont améliorées. En particulier, les inventeurs ont trouvé qu'en superposant au moins deux produits particuliers, la quantité de filtres UV liposolubles nécessaire pour obtenir une protection donnée est inférieure à la quantité de filtres liposolubles qu'il serait nécessaire d'incorporer dans une composition unique pour obtenir le même niveau de protection solaire.

La présente invention ne concerne pas le domaine des crèmes de protection solaires dont l'indice de protection est élevé et qui doivent pouvoir être appliquées facilement sur de grandes surfaces de peau. L'invention concerne plutôt le domaine des produits de soin et de maquillage, qui sont utilisés au quotidien par les consommateurs pour obtenir un effet cosmétique de soin et/ou de maquillage indépendant de celui d'une protection solaire.

Les inventeurs ont découvert de façon surprenante qu'il est possible de réaliser un soin cosmétique de la peau du corps ou du visage, ou un maquillage qui conserve ses fonctions premières de soin ou de maquillage sans dénaturation de ses propriétés sensorielles, tout en assurant une protection solaire satisfaisante. Le produit cosmétique de protection de l'invention permet également de réaliser un soin de peau ou un maquillage de la peau doté d'une texture et d'une sensorialité agréables qui assure en outre une protection vis-à-vis du rayonnement ultraviolet améliorée.

Ces effets sont obtenus en superposant au moins deux couches de produits différents, une première puis une deuxième composition, la deuxième composition comprenant essentiellement des poudres et au moins un filtre UV organique soluble dans chacun des phases huileuses des deux compositions et présentant un indice de protection solaire SPF inférieur ou égal à 10. L'application d'une composition sous forme de poudre sur le premier dépôt permet d'améliorer significativement la protection vis-à-vis des ultraviolets, par rapport à la protection que l'on obtiendrait par l'application d'une seule composition. Le produit cosmétique de l'invention permet l'obtention d'une protection solaire très élevée tout en appliquant des produits à la texture et à la sensorialité plaisantes, grâce à la superposition de deux produits qui sont par ailleurs dotés de fonctions cosmétiques propres indépendantes de la protection solaire.

La protection vis-à-vis des UV conférée par l'application successive des deux compositions du produit de l'invention, mesurée par exemple par le SPF, est supérieure à la protection conférée par l'application de chaque composition prise séparément. Elle est également supérieure à la somme des protections conférées par l'application séparée de chacune des deux compositions. Aussi, la superposition des deux compositions n'entraîne pas une simple addition des effets produits par chaque composition prise individuellement, mais produit bien un effet de synergie qui n'était pas prévisible par l'homme du métier.

### DESCRIPTION DE L'INVENTION

### Méthode d'application

Ainsi, la présente invention a pour objet un produit cosmétique de soin selon la revendication 1. Il peut être utilisé dans une méthode de protection de la peau contre les rayons UV. Une telle méthode utilise au moins deux compositions cosmétiques de soin et/ou de maquillage, et comprend:
- l'application sur au moins une partie de la peau du corps ou du visage, d'au moins une première couche d'une première composition cosmétique comprenant une phase huileuse, et une phase aqueuse, suivie de
- l'application sur ladite première couche, d'au moins une deuxième couche d'une deuxième composition anhydre comprenant au moins 70% en poids de poudres choisies parmi les charges et les pigments, et une phase huileuse,
ladite méthode étant caractérisée en ce que la seconde composition contient au moins un filtre UV organique soluble dans chacune des phases huileuses des deux compositions et présente un indice de protection solaire SPF inférieur ou égal à 10. Dans cette méthode, les deux compositions ne sont pas destinées à être mélangées préalablement à leur application, ou après leur application sur la peau. On cherche au contraire à superposer les dépôts de chaque composition sur la peau. Aussi, la deuxième composition est avantageusement appliquée à la surface du dépôt de la première composition sur la peau, et la personne qui applique le produit ne cherche pas - au moment de l'application de la deuxième composition sur la première - à détruire l'intégrité de la première couche afin de conserver l'effet cosmétique qui y est associé. Par exemple, en appliquant une poudre sur un fond de teint, la poudre ne doit pas détruire substantiellement le dépôt de fond de teint qui cache les imperfections de la peau. En particulier, les deux compositions ne contiennent pas d'ingrédients susceptibles de réagir chimiquement entre eux par la création de liaisons covalentes.

La première et la deuxième composition remplissent chacune indépendamment l'une de l'autre une fonction propre qui n'est pas altérée par la superposition de leurs dépôts. Elles peuvent à ce titre être utilisées seules tout en procurant un effet cosmétique recherché par le consommateur.

Selon la méthode décrite ci-dessus, le consommateur ou l'esthéticienne applique au moins deux dépôts de produits cosmétiques successifs qui conservent leur intégrité sur la peau. Le maquillage ou le soin formé sur la peau n'est pas sous la forme d'une seule couche homogène après l'application des deux compositions.

Selon la méthode décrite ci-dessus, l'effet protecteur global mesuré par la valeur du SPF après application de la première composition et de la seconde composition, est avantageusement supérieur à la plus haute valeur SPF de chacune desdites compositions ; il est aussi avantageusement supérieur à la somme des valeurs SPF de chacune desdites compositions.

### Filtre UV

Le filtre UV de la seconde composition est au moins un filtre UV organique liposoluble, éventuellement en mélange avec un filtre UV minéral.

Par "filtre UV organique", on entend tout composé organique absorbant un rayonnement UV dans la gamme de longueurs d'onde allant de 280 nm à 400 nm. Le filtre UV est de préférence « liposoluble » en ce sens qu'il peut être dissous à l'état moléculaire dans une huile, ou être dispersé dans une huile sous forme colloïdale ou sous forme micellaire.

### Filtres UV organiques liposolubles

Les filtres UV organiques liposolubles peuvent notamment être choisis parmi différentes familles de composés chimiques. On peut notamment citer les dérivés de l'acide para-aminobenzoïque, les dérivés salicyliques, les dérivés cinnamiques, les benzophénones et aminobenzophénones, les dérivés anthraniliques, les dérivés du dibenzoylméthane, les dérivés de [beta],[beta]'-diphénylacrylate, les dérivés du benzylidène camphre, les dérivés du phényl benzotriazole, les dérivés triazine, les bis-résorcinyl triazines, les dérivés d'imidazolines, les dérivés du benzalmalonate, les dérivés de 4,4-diarylbutadiène, les dérivés de benzoxazole, les mérocyanines et leurs mélanges.

Des exemples de dérivés de l'acide para-aminobenzoïque sont l'Ethyl PABA, l'Ethyl Dihydroxypropyl PABA et l'Ethylhexyl Diméthyl PABA. Des dérivés salicyliques sont notamment l'Homosalate vendu notamment sous le nom "Eusolex HMS^{®}" par Rona/EM Industries ; l'Ethylhexyl Salicylate vendu notamment sous le nom "NEO HELIOPAN OS^{®}" par SYMRISE ; le Dipropylèneglycol Salicylate vendu notamment sous le nom "DIPSAL^{®}" par SCHER ; ou le TEA Salicylate vendu notamment sous le nom "NEO HELIOPAN TS^{®}" par SYMRISE.

Parmi les dérivés salicyliques, on citera l'Homosalate vendu notamment sous le nom NEO HELIOPAN^{®} HMS; l'Ethylhexyl Salicylate vendu notamment sous le nom NEO HELIOPAN^{®} OS par SYMRISE.

Parmi les dérivés du cinnamate, on peut citer notamment de manière non limitative: l'éthyl-2-hexyl-p-methoxycinnamate, l'isopropyl-p-methoxycinnamate, Isoamyl Methoxycinnamate, le Cinoxate (2-éthoxyéthyl-p-methoxycinnamate), Diéthanolamine Methoxycinnamate, Glyceryl Ethyl-2-hexanoate Di-p-methoxycinnamate, [4-bis(trimethylsiloxy)methylsilyl-3-methylbutyl]-3,4,5,-trimethoxycinnamate.

Parmi les dérivés du cinnamate mentionnés ci-dessus, on utilisera tout particulièrement l'éthyl-2-hexyl-p-methoxycinnamate encore appelé Ethylhexyl Methoxycinnamate ou Octyl Methoxycinnamate (de dénomination USAN : Octinoxate) proposé à la vente sous les dénominations commerciales PARSOL MCX de la Société DSM NUTRITIONAL PRODUCTS, UVINUL MC 80 de la société BASF.

Parmi les dérivés de benzophénone, on citera la Benzophénone-1 vendu sous le nom commercial UVINUL^{®} 400; la Benzophénone-2 vendu sous le nom commercial UVINUL D50; la Benzophénone-3 ou Oxybenzone vendu sous le nom commercial UVINUL^{®} M40; la Benzophénone-6 vendu sous le nom commercial HELISORB 11; et la Benzophénone-8 vendu sous le nom commercial SPECTRASORB^{®} UV-24.

Une aminobenzophénone est par exemple le 2-(4-diéthylamino-2-hydroxybenzoyl)-benzoate de n-hexyle vendu notamment sous le nom commercial "UVINUL^{®} A +" par BASF.

Parmi les dérivés anthraniliques, on citera le menthylanthranilate vendu notamment sous la référence NEO HELIOPAN^{®} MA par SYMRISE.

Parmi les filtres UV dérivés du dibenzoylméthane, on peut notamment citer, de manière non limitative: le 2-méthyldibenzoylméthane, le 4-méthyldibenzoylméthane, le 4-isopropyldibenzoylméthane, le 4-tert-butyldibenzoylméthane, le 2,4-diméthyldibenzoylméthane, le 2,5-diméthyldibenzoylméthane, le 4,4'-diisopropyldibenzoylméthane, le 4,4'-diméthoxydibenzoylméthane, le 4-tert-butyl-4'-méthoxydibenzoylméthane, le 2-méthyl-5-isopropyl-4'-méthoxydibenzoylméthane, le 2-méthyl-5-tert-butyl-4'-méthxydibenzoylméthane, le 2,4-diméthyl-4'-méthoxydibenzoylméthane, le 2,6-diméthyl-4-tert-butyl-4'-méthoxydibenzoylméthane.

Parmi les dérivés du dibenzoylméthane mentionnés ci-dessus, on utilisera tout particulièrement le 4-(ter-butyl)-4'-méthoxy dibenzoylméthane encore appelé Butyl Methoxy Dibenzoylmethane (en abrégé BMDBM, de dénomination INCI 1-[4-(1,1-dimethylethyl)-phenyl]-3-(4-methoxyphenyl)-1,3-propanedione et de dénomination USAN Azobenzone) proposé à la vente sous les dénominations commerciales de PARSOL^{®} 1789 de la Société DSM NUTRITIONAL PRODUCTS, ou EUSOLEX^{®} 9020 de la société MERCK.

Deux dérivés du [beta],[beta]'-diphénylacrylate sont l'Octocrylène, vendu notamment sous le nom commercial "UVINUL^{®} N539" par BASF ; et l'Etocrylène, vendu notamment sous le nom commercial "UVINUL^{®} N35" par BASF.

Des exemples de dérivés du benzylidène camphre sont le 3-Benzylidène camphre ; le Méthylbenzylidène camphre vendu notamment sous le nom "EUSOLEX^{®} 6300" par MERCK ; et le Polyacrylamidométhyle Benzylidène Camphre.

On peut citer comme dérivés du phényl benzotriazole, le Drométrizole Trisiloxane vendu notamment sous le nom "Silatrizole^{®}" par RHODIA CHIMIE.

Parmi les dérivés de triazine ; on peut citer l'Ethylhexyl triazone vendu notamment sous le nom commercial "UVINUL^{®} T150" par BASF ; le Diéthylhexyl Butamido Triazone vendu notamment sous le nom commercial "UVASORB^{®} HEB" par SIGMA 3V ; le 2,4,6-tris(4'-amino benzalmalonate de dinéopentyle)-s-triazine ; le 2,4,6-tris(4'-amino benzalmalonate de diisobutyle)-s- triazine ; le 2,4-bis(4'-amino benzalmalonate de dinéopentyle)-6-(4'-aminobenzoate de n-butyle)-s-triazine ; le Bis-EthylhexyloxyphenolMethoxyphenyl-Triazine et le 2,4-bis(4'-amino benzoate de n-butyle)-6-(aminopropyltrisiloxane)-s-triazine.

Un dérivé bis-résorcinyl triazines est le Bis-Ethylhexyloxyphénol Méthoxyphenyl Triazine vendu notamment sous le nom commercial "TINOSORB^{®} S" par CIBA GEIGY.

Un dérivé d'imidazoline est l'Ethylhexyl Diméthoxybenzylidene Dioxoimidazoline Propionate.

Des dérivés du benzalmalonate sont les Polyorganosiloxanes à fonction benzalmalonate tels que le Polysilicone-15 vendu notamment sous la dénomination commerciale " PARSOL^{®} SLX " par DSM Nutritional Products, Inc. ; et le Di-neopentyl 4'-méthoxybenzalmalonate.

Un dérivé de benzoxazole est le 2,4-bis-[5-1(diméthylpropyl)benzoxazol-2-yl-(4-phenyl)-imino]-6-(2-ethylhexyl)-imino-1,3,5-triazine vendu notamment sous le nom d'UVASORB^{®} K2A par Sigma 3V.

Un dérivé de mérocyanine est l'Octyl-5-N,N-diéthylamino-2-phénysulfonyl-2,4-pentadiénoate.

Un exemple de dérivé de 4,4-diarylbutadiène est le 1,1-dicarboxy (2,2'-diméthyl-propyl)-4,4-diphénylbutadiène.

Dans le cadre de la présente invention, on choisit de préférence un filtre organique liposoluble parmi les filtres suivants et leurs mélanges: l'Ethylhexylsalicylate ; l'Octocrylène ; le Ethylhexyl triazone ; l'Ethylhéxyl Méthoxycinnamate ; le Butyl Méthoxydibenzoylméthane ; le 2-(4-diéthylamino-2-hydroxybenzoyl)-benzoate de n-héxyle ; le Drométrizole Trisiloxane ; le Méthylène bis-Benzotriazolyl Tétramethylbutylphénol ; la Bis-Ethylhexyloxyphénol Méthoxyphenyl Triazine ; la Benzophénone-3 ou l'Oxybenzone.

Les compositions peuvent contenir indépendamment l'une de l'autre un filtre UV de nature chimique identique ou différente.

### Filtres UV minéraux

Le filtre UV peut comprendre au moins un filtre UV minéral.

Par "filtre UV minéral", on entend tout composé minéral absorbant un rayonnement UV dans la même gamme de longueurs d'onde qu'indiquée précédemment.

Les filtres UV minéraux peuvent être choisis parmi des pigments d'oxydes métalliques de taille moyenne des particules généralement comprises entre 5 nm et 100 nm, de préférence entre 10 nm et 50 nm comme par exemple des pigments d'oxyde de titane (amorphe ou cristallisé sous forme rutile et/ou anatase), ou de zinc.

Les pigments peuvent être traités en surface ou non.

Les pigments traités en surface sont des pigments qui ont subi un ou plusieurs traitements de surface de nature chimique, électronique, mécanochimique et/ou mécanique avec des composés tels que décrits par exemple dans Cosmetics & Toiletries, Février 1990, Vol. 105, p. 53-64 , tels que des aminoacides, de la cire d'abeille, des acides gras, des alcools gras, des tensio-actifs anioniques, des lécithines, des sels de sodium, potassium, zinc, fer ou aluminium d'acides gras, des alcoxydes métalliques (de titane ou d'aluminium), du polyéthylène, des silicones, des protéines (collagène, élastine), des alcanolamines, des oxydes de silicium, des oxydes métalliques ou de l'hexamétaphosphate de sodium.

Les silicones utilisées pour le traitement des pigments sont par exemple choisies dans le groupe contenant les alkyl silanes, les polydialkylsiloxanes, et les polyalkylhydrogénosiloxanes.

Bien entendu, les pigments d'oxydes métalliques avant leur traitement par des silicones, peuvent avoir été traités par d'autres agents de surface, en particulier par de l'oxyde de cérium, de l'alumine, de la silice, des composés de l'aluminium, des composés du silicium, ou leurs mélanges.

Dans le cadre de l'invention on préfère utiliser l'oxyde de titane amorphe ou cristallisé sous forme rutile et/ou anatase comme filtre UV minéral.

La teneur en filtre UV minéral dans la première composition est par exemple inférieure ou égale à 25% en poids, avantageusement comprise entre 2 et 20% en poids, plus particulièrement comprise entre 3 et 10% en poids, par rapport au poids total de la première composition.

La teneur en filtre UV minéral dans la deuxième composition est par exemple inférieure ou égale à 25% en poids, avantageusement comprise entre 2 et 20% en poids, plus particulièrement comprise entre 3 et 10% en poids, par rapport au poids total de la deuxième composition.

Selon un mode de réalisation de l'invention, la première composition est exempte de filtre minéral et de filtre organique, et la deuxième composition comprend au moins un filtre organique soluble dans chacune des phases huileuses des deux compositions, et éventuellement au moins un filtre minéral.

Selon un autre mode de réalisation de l'invention, la première composition comprend au moins un filtre organique, et la deuxième composition comprend au moins un filtre organique soluble dans chacune des phases huileuses des deux compositions, et éventuellement au moins un filtre minéral.

La teneur en filtre UV dans la première composition est par exemple inférieure ou égale à 40 % en poids, avantageusement comprise entre 5 et 30% en poids, plus particulièrement comprise entre 10 et 20% en poids, par rapport au poids total de la première composition.

La teneur en filtre UV dans la deuxième composition est par exemple inférieure ou égale à 30% en poids, avantageusement comprise entre 1 et 20% en poids, plus particulièrement comprise entre 5 et 15 % en poids, par rapport au poids total de la deuxième composition.

Selon un mode de réalisation de l'invention, la première composition comprend :
- de 5 à 8% en poids de Méthoxycinnamate d'octyle par rapport au poids total de la première composition, et éventuellement de 0,5 à 3% en poids de Benzophénone-3 par rapport au poids total de la première composition, ou
- de 4 à 8% en poids de Méthoxycinnamate d'octyle par rapport au poids total de la première composition, et éventuellement au moins un filtre UV minéral, de préférence de 4,5 à 15% en poids d'un nanotitane par rapport au poids total de la première composition.

Selon un mode de réalisation de l'invention, la deuxième composition comprend de 4 à 8% en poids de Méthoxycinnamate d'octyle par rapport au poids total de la deuxième composition, et éventuellement au moins un filtre UV minéral, de préférence de 4,5 à 15% d'un nanotitane par rapport au poids total de la deuxième composition.

### Phases huileuses des deux compositions

La première composition contient au moins une phase huileuse, de préférence une seule phase huileuse. La deuxième composition contient également au moins une phase huileuse, de préférence une seule phase huileuse. Chacune desdites phases huileuses et/ou le mélange desdites phases huileuses solubilise le filtre UV organique liposoluble.

On entend par « phase huileuse » une huile ou un mélange d'huiles miscibles entre elles. Par « huile », on entend, au sens de l'invention, un corps gras, non soluble dans l'eau, liquide à 25°C et 0,1 MPa, et de préférence non volatile ayant une pression de vapeur, à 25°C et 0,1 MPa, non nulle inférieure à 2,6 Pa, de préférence inférieure à 0,13 Pa.

Dans un mode de mise en oeuvre, la première et la deuxième composition contiennent chacune une phase huileuse. Les deux phases huileuses des compositions sont de préférence miscibles entre elles.

On peut évaluer la miscibilité des deux phases huileuses entre elles selon le protocole suivant. Dans un bécher, on pèse la première phase huileuse puis on ajoute dans le bêcher la deuxième phase huileuse selon le rapport massique des phases huileuses de chaque formule. On agite pendant 5 minutes et puis on conditionne le tout dans un pilulier de 120 ml. On laisse reposer pendant 24 heures à 25°C. Après 24 heures de repos, si le mélange est visuellement limpide et homogène, on considère que les deux phases sont miscibles.

On préfère également que la phase huileuse de la première composition et la phase huileuse de la deuxième composition contienne des huiles miscibles entre elles. Le protocole d'évaluation de la miscibilité des huiles de la phase huileuse peut être identique à celui décrit précédemment.

La phase huileuse de la première composition et/ou la phase huileuse de la deuxième composition contiennent de préférence au moins une huile polaire. On entend par huile polaire, une huile qui contient au moins un, de préférence au moins deux atomes d'oxygène ou des doubles liaisons conjuguées.

L'huile est de préférence choisie parmi les mono- et di-esters aliphatiques, les esters aromatiques non hydroxylés, les carbonates aliphatiques et les silicones phénylés.

Par ester aliphatique, on entend un composé constitué d'atomes de carbone, d'atomes d'hydrogène et d'au moins un groupe carboxyle COO. Par monoester, on entend un composé comprenant un groupe COO, et par diester, on entend un composé comprenant deux groupes COO.

Par ester hydroxylé, on entend un composé comprenant au moins un groupe COO et au moins un groupe OH.

Dans un autre mode de réalisation, on préfère un ester choisi parmi les monoesters et les diesters aliphatiques.

Comme huiles polaires, on peut citer par exemple
- les mono-et di-esters aliphatiques, notamment i) les mono-esters d'un acide carboxylique aliphatique linéaire ou ramifié, saturé ou insaturé, de préférence saturé, comprenant de 8 à 20 atomes de carbone, et d'un mono-alcool aliphatique comprenant de 3 à 20 atomes de carbone, ii) les di-esters aliphatiques d'un di-acide aliphatique carboxylique comprenant de 4 à 10 atomes de carbone et d'un mono-alcool,
- les mono-esters de l'acide benzoïque et d'un alcool aliphatique comprenant de 8 à 20 atomes de carbone, le benzoate d'éthyle-2-hexyle, le benzoate d'octyl-2-dodécyle, le benzoate d'isostéaryle, le C12-C15 alkylbenzoate,
- les tri et tétra-esters tels que les esters du pentaérytritol, notamment le tétraisoséarate de pentaerythrityle, les esters du triméthylolpropane, notamment le triisoséarate de triméthylolpropane, les esters de l'acide citrique, notamment le citrate tridécyle, et le triméllitate de tridécyle,
- les di-alkyl-carbonates dont les groupes alkyles contiennent de 8 à 18 atomes de cabone tels que le dicaprylyl-carbonate, le di-(éthyl-2-hexyl)-carbonate,
- les mono ou di-esters aliphatiques hydroxylés tels que i) les esters d'un mono ou di-acide carboxylique aliphatique hydroxylé comprenant de 3 à 20 atomes de carbone, et d'un mono-alcool aliphatique aliphatique comprenant de 6 à 20 atomes de carbone, par exemple le lactate d'isostéaryle, l'hydroxystéarate d'octyle, l'hydroxystéarate d'octyldodécyle, le lactate de cétyle, le lactate de myristyle, le diisostéaryl-malate, ou ii) les mono et di-esters aliphatiques de polyol, en particulier de diols et de triols, tels que les esters d'un mono acide carboxylique aliphatique comprenant de 3 à 20 atomes de carbone, et d'un diol ou d'un triol aliphatique comprenant de 3 à 20 atomes de carbone,
- les mono- et di-esters hydroxylés aromatiques d'un acide carboxylique aromatique hydroxylé et d'un mono-alcool aliphatique comprenant au moins 10 atomes de carbone,
- les alcools aliphatiques saturés ou insaturés ayant de 8 à 26 atomes de carbone, comme l'octyldodécanol, l'octyldécanol, le 2-butyloctanol, le 2-hexyldécanol, le 2-undécylpentadécanol,
- les acides mono-carboxyliques aliphatiques saturés ou insaturés ayant de 7 à 29 atomes de carbone tels que l'acide oléique, l'acide linoléique, l'acide linolénique ou l'acide isostéarique,
- les huiles de silicone comportant au moins un groupement alcoxy ou phényle, pendant ou en bout de chaîne siliconée, ayant de 2 à 24 atomes de carbone, notamment la phényltriméthicone, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes;
- les glycols,
- les éthers aliphatiques comprenant plus de 10 atomes de carbone,
- les triglycérides, par exemple les huiles de tournesol, de maïs, de soja, de courge, de pépins de raisin, de sésame, de noisette, d'abricot, de macadamia, d'arara, de tournesol, de ricin, d'avocat, de jojoba, de beurre de karité, les triglycérides des acides caprylique/caprique, le triheptanoate de glycéryle, le trioctanoate de glycéryle, le tri-2-ethylhexanoate de glycéryle, le triisostéarate de glycéryle, le triisononanoate de glycéryle, le trimyristate de glycéryle,le triisopalmitate de glycéryle, et
- leurs mélanges.

Dans un mode de réalisation, on préfère que l'huile soit un ester choisi parmi les monoesters et les diesters aliphatiques.

Selon un autre mode de mise en oeuvre, l'huile est une huile siliconée comprenant des groupes carbonés aromatiques.

Parmi les mono- et di-esters aliphatiques, on préfère les esters comprenant de 10 à 25 atomes de carbone, de préférence de 14 à 22 atomes de carbone, par exemple les esters de l'acide isononanoïque tels que l'isononanoate d'isononyle, l'isononanoate d'isodécyle, l'isononanoate de décyl-2-héxyle, l'isononanoate d'isostéaryle, l'isononanoate de cétéaryle, l'isononanoate de tridécyle.

On peut citer aussi le myristate d'isopropyle, le palmitate d'éthyl-2-hexyle, le stéarate d'octyl-2-dodécyle, l'érucate d'octyl-2-dodécyle, l'isostéarate d'isostéaryle, le stearoyl-stearate d'octyl dodecyl, le palmitate d'isostéaryle, le stéarate d'isocétyle, le myristate d'octyldodécyle, le trilinoléate de triisostéaryle, le néodécanoate d'octyldodécyle, l'octyldodécyl octanoate, le stéarate d'isobutyle, la néopentanoate d'isodécyle, le néopentanoate d'octyldodécyle, l'iso-stéarate d'éhyl-2 héxyle, l'isostéarate de butyle, le palmitate d'isopropyle, l'heptanoate de stéaryle, le stéarate d'isopropyle, le néopentanoate d'isostéaryle, l'isostéarate d'isopropyle, l'octanoate de cétyle (ou octanoate de palmityle), le stéarate de butyle, le laurate d'hexyle (ou dodécanoate d'hexyle), le laurate d'éthyle, l'oléate de décyle, l'oléate d'oléyle, le myristate de myristyle, le diméthyl-octanoate d'hexyldécyle, l'isostearate d'isocétyle, le myristate de héxyl-2-décyle, le palmitate de heptyl-2-undécyle, le 2-éthyl-hexanoate de cétyle.

Parmi les esters de d'acides dicarboxyliques, on peut encore citer le sébaçate de di-(éthyl-2-hexyle), le sébaçate de di-isopropyle, le succinate de di-(éthyl-2-hexyle), l'adipate de di-(héxyl-2-décyle), l'adipate de di-(heptyl-2-undécyle).

Parmi les mono et di-esters de polyol, on peut citer les di-esters d'alkylèneglycol avec un acide aliphatique ayant de 6 à 20 atomes de carbone, tel que le dioctanoate de propylène glycol, le diheptanoate de néopentylglycol et le diisononanoate de diéthylèneglycol; le butylène glycol dicaprate/dicaprylate, le propylène glycol dicaprate/dicaprylate, le di-caprate de néopentyl glycol, le diéthylène glycol diisononanoate, le di-iso-stéarate de propylene glycol, le propylène glycol dipélargonate, le dioctanoate de propylène glycol, le di hepatnoate de néopentyl glycol, le tripropylène glycol dipivalate ; et les mono-alkanoates de glycéryle tel que l'heptanoate de glycéryle, l'octanoate de glycéryle, et le décanoate de glycéryle.

Les huiles polaires préférées de la première composition sont le C₁₂-C₁₅ alkylbenzoate et le néopentanoate d'isodécyle.

L'huile préférée de la deuxième composition est la phényl trimethicone ou le polyisobutène hydrogéné.

### Galénique de la première composition

La première composition est de préférence sous la forme d'une émulsion contenant une phase aqueuse et une phase huileuse. La phase aqueuse représente de préférence au moins 10% en poids, de préférence encore au moins 15% en poids du poids de la première composition.

La première composition peut contenir ou non au moins un filtre UV choisi parmi les filtres UV organiques et les filtres UV minéraux.

La première composition peut contenir un filtre UV organique liposoluble et éventuellement un filtre minéral.

La première composition est préférentiellement destinée à être appliquée sur le visage et se présente de préférence sous la forme d'une émulsion huile-dans-eau, eau-dans-huile ou d'un gel aqueux. La composition est par exemple sous la forme d'une crème de soin, d'une lotion, d'un sérum ou d'un fluide pour le visage, d'un fond de teint, d'un lait, ou d'une base de teint.

La première composition peut également comprendre au moins une matière colorante, avantageusement choisie parmi les pigments, les colorants liposolubles et les nacres. Les pigments peuvent être blancs ou colorés, minéraux et/ou organiques, enrobés ou non. On cite, parmi les pigments utilisables, le dioxyde de titane éventuellement traité en surface, les oxydes de zirconium, de zinc ou de cérium , ainsi que les oxydes de fer ou de chrome, le violet de manganèse, le bleu outremer, l'hydrate de chrome et le bleu ferrique, le noir de carbone, et les laques à de colorant, notamment les laques de baryum, strontium, calcium ou aluminium.

Les nacres peuvent être choisies parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

La première composition de l'invention peut également comprendre un ou plusieurs filtres UV organiques hydrophiles tels que la Benzophénone-4 vendu sous le nom commercial UVINUL^{®} MS40ou bien encore le Méthylène bis-Benzotriazolyl Tétramethylbutylphénol, vendu sous forme solide notamment sous le nom commercial "MIXXIM BB/100^{®}" par FAIRMOUNT CHEMICAL ou sous forme micronisé en dispersion aqueuse notamment sous le nom commercial "TINOSORB^{®} M" par CIBA SPECIALTY CHEMICALS.

La première composition de l'invention peut également comprendre tout additif usuellement utilisé en cosmétique tels qu'une charge solide, des antioxydants, des tensio-actifs, des conservateurs, des polymères filmogènes, des parfums, des agents actifs cosmétiques, comme par exemple des émollients, des hydratants, des vitamines, des agents anti-âge, des agents éclaircissants, et leurs mélanges.

L'indice de protection solaire de la première composition est de préférence supérieur à 10. Le facteur de protection solaire SPF de la première composition est par exemple compris entre 30 et 40.

### Galénigue de la deuxième composition

La deuxième composition contient au moins un filtre UV organique liposoluble.

La deuxième composition est par exemple sous la forme d'une poudre libre ou compactée qui présente un indice de protection solaire mesurée selon la méthode du SPF inférieur ou égal à 10, et de préférence supérieur à 3. La deuxième composition a par exemple un SPF de l'ordre de 4.

La deuxième composition contient avantageusement le mélange d'au moins un filtre soluble dans chacune des phases huileuses des deux compositions et d'au moins filtre minéral.

La deuxième composition est préférentiellement destinée à être appliquée sur le visage et se présente de préférence sous la forme d'une poudre libre ou compactée. Elle contient de préférence au moins 80%, plus préférentiellement au moins 90% en poids de poudres choisies parmi les charges et les pigments couramment utilisés en cosmétique.

La deuxième composition est de préférence anhydre, en ce sens qu'elle contient moins de 10% en poids, de préférence moins de 5% en poids, de préférence encore moins de 3% en poids du poids de la composition.

La deuxième composition contient avantageusement des charges et des pigments, qui peuvent être choisis parmi les pigments minéraux, les pigments organiques et les pigments nacrés.

Les pigments minéraux peuvent être choisis parmi les oxydes de fer, en particulier les oxydes de fer noir, jaune, rouge et brun; le violet de manganèse; le bleu outremer; les oxydes de chrome, en particulier l'oxyde de chrome hydraté, le bleu ferrique, le noir de carbone, et leurs mélanges.

Parmi les pigments organiques, on peut citer notamment les laques obtenues à partir de colorants tels que les colorants D&C Black No. 2, FD&C Blue No. 1, FD&C Green No. 3, D&C Green No. 5, D&C Orange No. 4, D&C Orange No. 5, D&C orange No. 10, D&C No. red 3, D&C Red No. 6, D&C Red No. 7, D&C red No. 9, D&C red No. 13, D&C red No. 19, D&C Red No. 21, D&C Red No. 22, D&C Red No. 27, D&C Red No. 28, D&C Red No. 30, D&C Red No. 33, D&C Red No. 36, FD&C Red No. 40, FD&C Yellow No. 5, FD&C Yellow No. 6, D&C Yellow No. 10 et le carmin de cochenille.

Les pigments nacrés sont par exemple choisis parmi le mica recouvert d'oxyde de titane, le mica-titane recouvert d'oxyde de fer, le mica-titane recouvert de bleu ferrique, le mica-titane recouvert d'oxyde de chrome, le mica-titane recouvert d'un pigment organique tel que décrit précédemment, ainsi que les pigments à base d'oxychlorure de bismuth.

Les charges peuvent être minérales ou organiques, et de toutes formes, plaquettaires, sphériques ou oblongues.

Les charges sont choisies notamment parmi des charges inorganiques telles que:
- le talc, de préférence sous forme de particules généralement de dimensions inférieures à 40 µm;
- les micas d'origine naturelle ou synthétique ayant des dimensions de 2 à 200 µm, de préférence de 5 à 70 µm et une épaisseur de 0,1 à 5 µm, de préférence de 0,2 à 3 µm;
- le kaolin ayant des tailles de particules généralement inférieures à 30 µm;
- les savons métalliques dérivés d'acides organiques carboxyliques ayant de 8 à 22 atomes de carbone, de préférence de 12 à 18 atomes de carbone, par exemple le stéarate de zinc, le stéarate de magnésium ou le stéarate de lithium, le laurate de zinc, le myristate de magnésium, de préférence sous la forme de particules ayant des dimensions inférieures à 10 µm;
- les oxydes de zinc, les oxydes de titane; le carbonate de calcium; le carbonate de magnésium, l'hydrocarbonate de magnésium, la silice, les billes de verre, les billes de céramique;
- et leurs mélanges.

On peut aussi utiliser comme charges des charges organiques telles que:
- des amidons réticulés ou non, par exemple des amidons de maïs, de blé, ou de riz;
- des poudres de polymères de synthèse, réticulées ou non, sphéronisées ou non, expansées ou non, comme des poudres de polyéthylène, des poudres de polyester (par exemple isophtalate ou téréphtalate), des poudres de polyamide (par exemple des poudres de poly-β-alanine et des poudres de nylon comme celles commercialisées sous la dénomination ORGASOL^{®}), des poudres d'acide poly(méth)acrylique ou de poly(méth)acylate telles que les poudres de méthacrylate de méthyle réticulé, des poudres de polyuréthane telles que les copolymères de diisocyanate d'hexaméthylène et de triméthylol hexyl lactone vendus sous les dénominations PLASTIC POWDER^{®} D-400 et PLASTIC POWDER^{®} D-800 par la société TOSHIKI, des poudres de polystyrène réticulé par le divinylbenzène, des poudres de résine de silicone telles que des silsesquioxanes, ou des poudres de tétrafluoroéthylène (Téflon^{®}) ;
- et leurs mélanges.

La deuxième composition peut avoir divers usages cosmétiques, et notamment servir comme fond de teint, base de teint, poudre de maquillage du corps, poudre de soin, poudre de maquillage du visage, blush ou fard à joues, fard à paupières, enlumineur de teint, poudre de soleil, ou poudre de teint protectrice.

La composition de l'invention est de préférence une poudre libre de maquillage.

L'application de la deuxième composition peut être réalisée au pinceau, en spray, à l'éponge ou au doigt. Elle est de préférence réalisée au pinceau.

### Définition et mesure de l'indice de protection solaire

Le niveau de protection solaire conféré par chaque composition aussi que par la superposition des deux dépôts des compositions peut être mesuré par diverses méthodes connues de l'homme du métier, in vivo ou in vitro.

L'indice de protection solaire d'une composition peut être mesuré selon la méthode de mesure in vivo du facteur de protection solaire (SPF pour « Sun Protection Factor ») publiée par le Colipa, le CTFA SA, le JCIA et le CTHA en mai 2006 (sur le site http://www.colipa.eu/publications-colipa-the-european-cosmetic-cosmetics-association ; « International Sun Protection Factor Test Method - 2006 »). Selon cette méthode, le SPF d'une composition est défini comme le rapport entre le temps d'irradiation nécessaire pour atteindre le seuil érythématogène de la peau sur laquelle a été appliquée la composition, et le temps nécessaire pour atteindre le seuil érythématogène de la peau nue. La méthode publiée par le Colipa précise les conditions minimales à respecter pour que la mesure du SPF soit reproductible et significative. Les directives mentionnent notamment quelle quantité de composition doit être appliquée sur la peau, et quelle lampe d'irradiation doit être utilisée.

Il existe également d'autres méthodes permettant de quantifier le niveau de protection conféré par un produit cosmétique, comme par exemple la méthode PPD (Persistent Pigment Darkening), qui mesure la couleur de la peau observée 2 à 4 heures après une exposition de la peau aux UV-A (longueurs d'onde comprises entre de 320 nm à 400 nm). Cette méthode est utilisée depuis 1996 par la Japanese Cosmetic Industry Association (JCIA) pour l'étiquetage UV-A des produits et par les laboratoires de tests en Europe et aux Etats-Unis (Japan Cosmetic Industry Association - Technical Bulletin - Measurement Standards for UVA protection efficacy - Issued November 21, 1995 and effective of January 1, 1996). Le facteur de protection UVAPPD (FP UVAPPD) correspond au rapport i) de la dose de rayonnement UV-A nécessaire pour que la peau recouverte de composition cosmétique atteigne le seuil de pigmentation (MPPDp) à ii) la dose de rayonnement UV-A nécessaire pour que la peau nue atteigne le seuil de pigmentation (MPPDnp).

Dans le cadre de l'invention, on préfère mesurer le niveau de protection solaire que fournit la première composition, la deuxième composition et leur superposition en utilisant une méthode reprenant le protocole et les paramètres préconisés dans la méthode décrite par le Colipa en 2006, à la seule exception de la dose appliquée que l'on choisit égale à 0,8 mg/cm².

### Produit

La présente invention a donc pour objet un produit cosmétique comprenant au moins deux compositions cosmétiques conditionnées de façon séparée, dans un même emballage, telle que défini à la revendication 1. La seconde composition présente un SPF inférieur ou égal à 10.

La première composition est par exemple une crème de soin, une base de soin ou un fond de teint, tandis que la deuxième est une poudre.

Selon un mode de réalisation préféré, la première composition est une émulsion eau-dans-huile ou huile-dans-eau, qui contient ou non des pigments.

La première composition peut comprendre un ou plusieurs principes actifs choisis parmi les actifs hydratants, les actifs anti-âge, les actifs antioxydants, les actifs blanchissants et les actifs dépigmentants.

La deuxième composition est avantageusement une poudre libre, pressée ou compactée.

### Utilisation d'une poudre pour augmenter le SPF d'une crème

La présente demande présente également l'utilisation non revendiquée d'une deuxième composition cosmétique comprenant au moins 70% en poids de poudres choisies parmi les charges et les pigments, une phase huileuse et au moins un filtre UV organique liposoluble, pour son application sur une première composition ne contenant pas de filtre UV afin d'obtenir un résultat cosmétique aux propriétés sensorielles améliorées tout en obtenant un niveau de protection quotidien de la peau contre les rayons UV satisfaisant grâce à l'application successive de la première puis de la deuxième composition sur la peau.

### Méthode d'augmentation du SPF

La présente demande présente également une méthode non revendiquée pour augmenter l'indice de protection solaire d'une première composition cosmétique contenant une phase huileuse, et éventuellement un ingrédient actif, tout en préservant ses qualités sensorielles, laquelle méthode consiste à appliquer la première composition sur la peau pour former une couche, puis à appliquer sur cette couche une deuxième composition contenant une phase huileuse, au moins 70% en poids de charges et de pigments, et au moins un filtre UV organique liposoluble.

Le filtre UV organique liposoluble est de préférence soluble dans le mélange des deux phases huileuses des deux compositions. Il peut être conforme à la description qui en a été faite plus haut.

Selon un mode de réalisation, la deuxième composition comprend au moins un filtre UV organique liposoluble et éventuellement au moins un filtre UV minéral.

La première composition peut comprendre ou ne pas comprendre de filtre UV.

Les caractéristiques qui ont été décrites précédemment en rapport avec la première composition et la deuxième composition s'appliquent à l'utilisation d'une poudre pour augmenter le SPF d'une crème telle que décrite précédemment et à la méthode d'augmentation du SPF décrite ci-dessus. Dans les exemples, tous les pourcentages sont donnés en poids, sauf indication contraire, et la température est exprimée en degré Celsius sauf indication contraire, et la pression est la pression atmosphérique, sauf indication contraire.

### EXEMPLE : Mesure de l'indice de protection solaire résultant de la méthode de l'invention

La mise en oeuvre de l'invention nécessite deux compositions distinctes qui sont décrites successivement ci-après :

### Premières compositions

On a préparé les produits suivants.
- La BASE DE SOIN 1 contient 3% de Benzophénone-3, 7.5% de Méthoxycinnamate d'octyle et 5% de nanotitane traité. Son SPF mesuré sur 10 sujets selon le protocole et les conditions de mesure données dans la méthode « International Sun Protection Factor Test Method - 2006 » publiée par le Colipa est égal à 36.4 +/- 4.8.
- La BASE DE SOIN 2 ne contient pas de filtres UV.
- Le premier fond de teint liquide (FOND DE TEINT 1) contient 6% de Méthoxycinnamate d'octyle et 6% de nanotitane traité. La valeur de SPF mesuré sur 10 sujets selon le protocole et les conditions de mesure données dans la méthode « International Sun Protection Factor Test Method - 2006 » publiée par le Colipa est égal à 36.3 +/- 5.0.
- Le deuxième fond de teint (FOND DE TEINT 2) ne contient pas de filtres UV.

Leur composition détaillée est donnée ci-après.

| **BASE DE SOIN 1** | |
|---|---|
| Dénomination INCI | % en poids |
| EAU | qsp 100 |
| C12-15 ALKYL BENZOATE | 20,3 |
| METHYL TRIMETHICONE | 9,0 |
| ETHYLHEXYL METHOXYCINNAMATE | 7,5 |
| NANOTITANE TRAITE | 5,0 |
| BUTYLENE GLYCOL | 5,0 |
| ETHYLENE/ACRYLIC ACID COPOLYMER | 5,0 |
| GLYCEROL | 4,3 |
| CETYL PEG/PPG-10/1 DIMETHICONE | 4,0 |
| BENZOPHENONE-3 | 3,0 |
| PHENOXYETHANOL | 0,85 |
| PIGMENTS ET NACRES | 8,7 |
| CAPRYLYL GLYCOL | 0,5 |
| ACIDE POLYHYDROXYSTEARIQUE | 0,45 |
| EXTRAIT DE BETULA ALBA JUICE | 0,4 |
| EDTA TETRASODIQUE | 0,2 |
| KHARISMAL | 0,1 |
| STEARYL GLYCYRRHETINATE | 0,1 |

| **BASE DE SOIN 2** | |
|---|---|
| Dénomination INCI | % en poids |
| C12-15 ALKYL BENZOATE | 35,5 |
| EAU | qsp 100 |
| METHYL TRIMETHICONE | 9,0 |
| BUTYLENE GLYCOL | 5,1 |
| ETHYLENE/ACRYLIC ACID COPOLYMER | 5,0 |
| GLYCEROL | 4,3 |
| CETYL PEG/PPG-10/1 DIMETHICONE | 4,0 |
| PHENOXYETHANOL | 0,8 |
| PIGMENTS ET NACRES | 8,6 |
| CAPRYLYL GLYCOL | 0,5 |
| EXTRAIT DE BETULA ALBA | 0,4 |
| EDTA TETRASODIQUE | 0,2 |
| KHARISMAL | 0,1 |
| STEARYL GLYCYRRHETINATE | 0,1 |
| BENZYL ACETATE | < 0,1 |
| EXTRAIT DE MAUVE (MALVA SYLVESTRIS) | < 0,1 |

| **FOND DE TEINT 1** | |
|---|---|
| Dénomination INCI | % en poids |
| EAU | qsp 100 |
| CYCLOPENTASILOXANE | 21,6 |
| NANOTITANE TRAITE | 7,0 |
| ETHANOL | 9,4 |
| ETHYLHEXYL METHOXYCINNAMATE | 6,0 |
| CETYL PEG/PPG-10/1 DIMETHICONE | 3,5 |
| ASCORBYL GLUCOSIDE | 2,1 |
| ACRYLATES / DIMETHICONE COPOLYMER | 2,0 |
| ISODECYL NEOPENTANOATE | 1,7 |
| PIGMENTS ET NACRES | 12,0 |
| BORON NITRIDE | 1,5 |
| CYCLOHEXASILOXANE | 1,1 |
| SORBITAN SESQUIOLEATE | 1,0 |
| SODIUM CITRATE | 0,5 |
| DISTEARDIMONIUM HECTORITE | 0,4 |
| PHENOXYETHANOL | 0,3 |
| HYDROXIDE DE SODIUM | 0,2 |
| EDTA TETRASODIQUE | 0,2 |
| EXTRAIT DE BETULA ALBA | 0,2 |
| KHARISMAL | 0,1 |
| PROPYLENE CARBONATE | 0,1 |

| **FOND DE TEINT 2** | |
|---|---|
| Dénomination INCI | % en poids |
| EAU | qsp 100 |
| CYCLOPENTASILOXANE | 21,6 |
| ISODECYL NEOPENTANOATE | 14,8 |
| PIGMENTS ET NACRES | 12,0 |
| ETHANOL | 9,4 |
| CETYL PEG/PPG-10/1 DIMETHICONE | 3,5 |
| ASCORBYL GLUCOSIDE | 2,1 |
| ACRYLATES / DIMETHICONE COPOLYMER | 2,0 |
| BORON NITRIDE | 1,5 |
| CYCLOHEXASILOXANE | 1,1 |
| SORBITAN SESQUIOLEATE | 1,0 |
| CITRATE DE SODIUM | 0,5 |
| DISTEARDIMONIUM HECTORITE | 0,4 |
| PHENOXYETHANOL | 0,3 |
| HYDROXYDE DE SODIUM | 0,2 |
| TETRASODIUM EDTA | 0,2 |
| EXTRAIT DE BETULA ALBA | 0,2 |
| KHARISMAL | 0,1 |
| PROPYLENE CARBONATE | 0,1 |
| BENZYL ACETATE | < 0,1 |

### Deuxièmes compositions

On a préparé les compositions suivantes.
- La POUDRE 1 contient 5% de Méthoxycinnamate d'octyle et 4,5% de nanotitane traité. Son SPF mesuré sur 10 sujets selon le protocole et les conditions de mesure données dans la méthode « International Sun Protection Factor Test Method - 2006 » publiée par le Colipa est égal à 6.3 +/- 1.1.
- La POUDRE 2 contient 9.5% de Méthoxycinnamate d'octyle.

Leur composition détaillée est donnée ci-après.

| **POUDRE 1** | |
|---|---|
| Dénomination INCI | % en poids |
| TALC | qsp 100 |
| FLUORPHLOGOPITE SYNTHETIQUE | 25,3 |
| SILICE | 12 |
| ETHYLENE/ACRYLIC ACID COPOLYMER | 5,0 |
| ETHYLHEXYL METHOXYCINNAMATE | 5,0 |
| NANOTITANE TRAITE | 4,5 |
| BORON NITRIDE | 4,0 |
| MAGNESIUM ASCORBYL PHOSPHATE | 3,2 |
| PIGMENTS ET NACRES | 7,2 |
| POLYMETHYL METHACRYLATE | 2,0 |
| PENTYLENE GLYCOL | 1,0 |
| C9-13 FLUOROALCOOL | 0,9 |
| EAU | 0,85 |
| ACIDE STEARIQUE | 0,6 |
| SODIUM DEHYDROACETATE | 0,45 |
| EDTA TETRASODIQUE | 0,2 |
| DIMETHICONE/METHICONE COPOLYMER | 0,2 |
| ACIDE PHOSPHORIQUE | 0,1 |
| HYDROXIDE D'ALUMINUM | 0,1 |
| ACIDE SORBIQUE | 0,1 |
| POLYISOBUTENE HYDROGENE | 0,1 |

| **POUDRE 2** | |
|---|---|
| Dénomination INCI | % en poids |
| TALC | qsp 100 |
| FLUORPHLOGOPITE SYNTHETIQUE | 25,3 |
| PIGMENTS ET NACRES | 7,2 |
| SILICE | 12 |
| ETHYLHEXYL METHOXYCINNAMATE | 9,5 |
| ETHYLENE/ACRYLIC ACID COPOLYMER | 5,0 |
| BORON NITRIDE | 4,0 |
| MAGNESIUM ASCORBYL PHOSPHATE | 3,2 |
| POLYMETHYL METHACRYLATE | 2,0 |
| PENTYLENE GLYCOL | 1,0 |
| EAU | 0,85 |
| DEHYDROACETATE DE SODIUM | 0,45 |
| ISOPROPYL TITANIUM TRIISOSTEARATE | 0,3 |
| TETRASODIUM EDTA | 0,2 |
| DIMETHICONE/METHICONE COPOLYMER | 0,15 |
| HYDROXYDE D'ALUMINUM | 0,1 |
| ACIDE SORBIQUE | 0,1 |
| POLYISOBUTENE HYDROGENE | 0,1 |

L'étude a porté sur 10 sujets.

Le protocole et les conditions de mesure de l'indice de protection solaire sont celles données dans la méthode « International Sun Protection Factor Test Method - 2006 » publiée par le Colipa, à l'exception des paramètres suivants :
- dose appliquée (0,8 mg/cm²).
- laps de temps entre l'application du produit et la mesure (5 min)

Dans la mesure où le protocole diffère ici de la méthode de mesure « International Sun Protection Factor Test Method - 2006 » publiée par le Colipa, on ne fait plus référence au SPF, mais à un indice de protection solaire, pour caractériser la protection conférée vis-à-vis des UV par ces compositions.

Lorsque l'on superpose les produits, on laisse sécher chaque produit 5 minutes avant d'appliquer le suivant.

Le tableau 1 donne les indices de protection mesurés pour chaque composition, selon le protocole précisé ci-dessus.

**Tableau 1 : Mesure de l'indice de protection solaire des compositions mises en oeuvre pour l'étude**

| Composition testée | Indice de protection |
|---|---|
| BASE DE SOIN 1 | 12,2 +/- 2,0 |
| BASE DE SOIN 2 | 2,1 +/- 0,2 |
| POUDRE 1 | 4,0 +/- 0,7 |
| POUDRE 2 | 4,0 +/- 0,7 |
| FOND DE TEINT 1 | 16,4 +/- 2,5 |
| FOND DE TEINT 2 | 3,6 +/- 0,5 |

Le tableau 2 donne les indices de protections mesurés selon le protocole ci-dessus (sauf mention contraire dans les tableaux), après application successive sur la peau, de deux ou trois compositions testées, selon l'ordre précisé dans le tableau.

**Tableau 2 : Mesure de l'indice de protection solaire de la superposition de deux ou trois compositions selon l'invention**

| Etapes de mise en oeuvre | Indice de protection |
|---|---|
| Superposition de la BASE DE SOIN 1, du FOND DE TEINT 1 puis de la POUDRE 1 | 41,9 +/- 5,0 |
| *Essai comparatif 1* | *29,6 +*/*- 3,4* |
| *Superposition de la BASE DE SOIN 1 puis du FOND DE TEINT 1* | |
| *Essai comparatif 2* | *25*,*3 +*/*- 4*,*1* |
| *Mélange des trois produits (BASE DE SOIN 1, FOND DE TEINT 1 ET POUDRE 1) appliqué en une couche de 2,4 mg*/*cm²* | |
| Superposition de la BASE DE SOIN 1 puis de la POUDRE 1 | 19,4 +/- 3,2 |
| Superposition de la BASE DE SOIN 2 puis de la POUDRE 2 | 8,1 +/- 1,4 |
| Superposition du FOND DE TEINT 1 puis de la POUDRE 1 | 31,5 +/- 4,1 |
| Superposition de la BASE DE SOIN 2 puis de la POUDRE 1 | 10,2 +/- 1,6 |
| Superposition du FOND DE TEINT 2 puis de la POUDRE 1 | 11,0 +/- 1,4 |

### Résultats

Le dépôt du mélange des trois produits (base de soin, fond de teint liquide et poudre), a un indice de protection solaire inférieur à celui du dépôt formé par la superposition des trois produits pris séparément. Le mélange des trois produits est très visqueux et n'est pas doté de propriétés sensorielles satisfaisantes permettant son application sur la peau. L'indice de protection solaire du dépôt résultant de la superposition des trois produits est donc supérieur à la fois i) à la somme des indices de protection solaire des dépôts de chacun des produits appliqués individuellement et ii) à l'indice de protection solaire du dépôt du mélange des trois produits, à quantité de dépôts égales.

La méthode de l'invention permet d'améliorer la protection de la peau vis-à-vis des UV tout en améliorant les propriétés sensorielles du produit cosmétique.

L'indice de protection solaire obtenu par la superposition des deux produits selon la méthode de l'invention est statistiquement significativement supérieur à la somme des deux indices de protections solaires individuels.

## Revendications

1. Produit cosmétique de soin de la peau comprenant au moins deux compositions cosmétiques conditionnées de façon séparée dans un même emballage,
- la première composition cosmétique étant destinée à être appliquée sur la peau et comprenant une phase aqueuse et une phase huileuse, et
- la seconde composition étant destinée à être appliquée sur la première composition et comprenant au moins 70% en poids de poudres choisies parmi les charges et les pigments et une phase huileuse,
**caractérisé en ce que** la seconde composition contient au moins un filtre UV organique soluble dans chacune des phases huileuses des deux compositions, et présente un indice de protection solaire SPF inférieur ou égal à 10.

2. Produit cosmétique selon la revendication 1, **caractérisé en ce que** la première composition présente un indice de protection solaire SPF supérieur à 10.

3. Produit cosmétique selon l'une des revendications 1 à 2, **caractérisé en ce que** la première composition est une crème de soin, une base de soin ou un fond de teint, tandis que la deuxième est une poudre.

4. Produit cosmétique selon l'une des revendications 1 à 3, **caractérisé en ce que** la première composition est une émulsion eau-dans-huile ou huile-dans-eau, qui contient ou non des pigments.

5. Produit cosmétique selon l'une des revendications 1 à 4, **caractérisé en ce que** la première composition est une composition comprenant un ou plusieurs principes actifs choisis parmi les actifs hydratants, les actifs anti-âge, les actifs antioxydants, les actifs blanchissants et les actifs dépigmentants.

6. Produit cosmétique selon l'une des revendications 1 à 5, **caractérisée en ce que** la deuxième composition est une poudre libre, pressée ou compactée.

7. Produit cosmétique selon l'une des revendications 1 à 6, **caractérisée en ce que** la première composition ne contient pas de filtre UV.

8. Produit cosmétique selon l'une des revendications 1 à 7 destiné à être utilisé dans le traitement de la protection de la peau contre les rayons UV.

## Patentansprüche

1. Kosmetisches Produkt zur Hautpflege, umfassend wenigstens zwei kosmetische Zusammensetzungen, die in einer gleichen Verpackung getrennt verpackt sind,
- wobei die erste kosmetische Zusammensetzung dazu bestimmt ist, auf die Haut aufgetragen zu werden, und eine wässrige Phase und eine ölige Phase umfasst, und
- wobei die zweite Zusammensetzung dazu bestimmt ist, auf die erste Zusammensetzung aufgetragen zu werden, und wenigstens 70 Gew.-% Pulver, ausgewählt aus den Füllstoffen und den Pigmenten, und eine ölige Phase umfasst,
**dadurch gekennzeichnet, dass** die zweite Zusammensetzung wenigstens einen organischen UV-Filter, der in jeder der öligen Phasen der beiden Zusammensetzungen löslich ist, enthält und einen Lichtschutzfaktor SPF von weniger als oder gleich 10 aufweist.

2. Kosmetisches Produkt nach Anspruch 1, **dadurch gekennzeichnet, dass** die erste Zusammensetzung einen Lichtschutzfaktor SPF von mehr als 10 aufweist.

3. Kosmetisches Produkt nach einem der Ansprüche 1 bis 2, **dadurch gekennzeichnet, dass** die erste Zusammensetzung eine Pflegecreme, eine Pflegegrundierung oder ein Make-up ist, während die zweite ein Puder ist.

4. Kosmetisches Produkt nach einem der Ansprüche 1 bis 3, **dadurch gekennzeichnet, dass** die erste Zusammensetzung eine Waser-in-Öl- oder Öl-in-Wasser-Emulsion ist, die Pigmente enthält oder nicht.

5. Kosmetisches Produkt nach einem der Ansprüche 1 bis 4, **dadurch gekennzeichnet, dass** die erste Zusammensetzung eine Zusammensetzung mit einem oder mehreren Wirkstoffen ist, die aus den hydratisierenden Wirkstoffen, den Anti-Aging-Wirkstoffen, den Antioxidationswirkstoffen, den Aufhellungswirkstoffen und den Depigmentierungswirkstoffen ausgewählt sind.

6. Kosmetisches Produkt nach einem der Ansprüche 1 bis 5, **dadurch gekennzeichnet, dass** die zweite Zusammensetzung ein ungebundener, gepresster oder verdichteter Puder ist.

7. Kosmetisches Produkt nach einem der Ansprüche 1 bis 6, **dadurch gekennzeichnet, dass** die erste Zusammensetzung keinen UV-Filter enthält.

8. Kosmetisches Produkt nach einem der Ansprüche 1 bis 7, das dazu bestimmt ist, bei der Behandlung des Schutzes der Haut vor UV-Strahlen verwendet zu werden.

## Claims

1. A cosmetic skincare product comprising at least two cosmetic compositions packaged separately in the same packaging,
- the first cosmetic composition being intended to be applied to the skin and comprising an aqueous phase and an oil phase, and
- the second composition being intended to be applied on the first composition and comprising at least 70% by weight of powders selected from fillers and pigments, and an oil phase,
**characterized in that** the second composition contains at least one organic UV-screening agent soluble in each of the oil phases of the two compositions, and having a sun protection index SPF of less than or equal to 10.

2. The cosmetic product as claimed in claim 1, **characterized in that** the first composition has a sun protection index SPF of greater than 10.

3. The cosmetic product as claimed in either of claims 1 and 2, **characterized in that** the first composition is a care cream, a care base or a foundation, while the second is a powder.

4. The cosmetic product as claimed in one of claims 1 to 3, **characterized in that** the first composition is a water-in-oil or oil-in-water emulsion which optionally contains pigments.

5. The cosmetic product as claimed in one of claims 1 to 4, **characterized in that** the first composition is a composition comprising one or more active ingredients selected from moisturizing active ingredients, anti-aging active ingredients, antioxidant active ingredients, whitening active ingredients and depigmenting active ingredients.

6. The cosmetic product as claimed in one of claims 1 to 5, **characterized in that** the second composition is a loose, pressed or compact powder.

7. The cosmetic product as claimed in one of claims 1 to 6, **characterized in that** the first composition does not contain any UU-screening agent.

8. The cosmetic product as claimed in one of claims 1 to 7, being intended to be used in the treatment for protection of the skin against UV rays.
